(19) Europäisches Patentamt / European Patent Office / Office européen des brevets

(11) **EP 4 342 875 A1**

(12) **EUROPEAN PATENT APPLICATION**
published in accordance with Art. 153(4) EPC

(43) Date of publication:
**27.03.2024   Bulletin 2024/13**

(21) Application number: **22804050.7**

(22) Date of filing: **20.05.2022**

(51) International Patent Classification (IPC):
**C07C 45/42** (2006.01)      **C07C 49/12** (2006.01)

(52) Cooperative Patent Classification (CPC):
**C07C 45/42; C07C 49/12**

(86) International application number:
**PCT/CN2022/094002**

(87) International publication number:
**WO 2022/242730 (24.11.2022 Gazette 2022/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.05.2021   CN 202110559517
21.05.2021   CN 202110557880**

(71) Applicants:
• **China Petroleum & Chemical Corporation
Beijing 100728 (CN)**
• **Shanghai Research Institute of Petrochemical
Technology, SINOPEC
Shanghai 201208 (CN)**

(72) Inventors:
• **YANG, Weimin
Shanghai 201208 (CN)**

• **LI, Xiangcheng
Shanghai 201208 (CN)**
• **WANG, Zhendong
Shanghai 201208 (CN)**
• **HAN, Xiao
Shanghai 201208 (CN)**
• **FENG, Xinqiang
Shanghai 201208 (CN)**
• **YUAN, Zhiqing
Shanghai 201208 (CN)**
• **QIAO, Jian
Shanghai 201208 (CN)**
• **LIU, Chuang
Shanghai 201208 (CN)**
• **LAN, Dawei
Shanghai 201208 (CN)**

(74) Representative: **Hoffmann Eitle
Patent- und Rechtsanwälte PartmbB
Arabellastraße 30
81925 München (DE)**

(54) **METHOD FOR PREPARING 2, 5-HEXANEDIONE BY CATALYZING AND CONVERTING BIOMASS BY ONE-POT SYNTHESIS**

(57)   The present invention discloses a biphasic solvent system for converting biomass to prepare 2,5-hexanedione and a one-pot process for catalytically converting biomass to prepare 2,5-hexanedione with said biphasic solvent system. The process comprises: contacting and reacting a biomass raw material with a hydrogenation catalyst using hydrogen gas as a hydrogen source in a heterogeneous system formed from an organic solvent, an inorganic salt and water to obtain 2,5-hexanedione; the hydrogenation catalyst comprises a hydrogenation active component and a support, wherein said support is selected from one or more of hydrophobic active carbon and graphene. The process of the present invention can achieve efficient conversion of biomass without the participation of acid catalysts, and have a very high selectivity for the product 2,5-hexanedione.

Fig. 1

EP 4 342 875 A1

**Description**

**Technical Field**

The present invention relates to the field of catalytic chemistry, and in particular, to a process for catalytically converting biomass to prepare 2,5-hexanedione.

**Background technology**

In recent years, with the rapid consumption of global fossil resources, the preparation of platform compounds and biofuels from biomass has become a hotspot in current studies. Among the many platform compounds prepared from biomass, 2,5-hexanedione (HDO) has a wide range of potential uses. It is widely used in medicine, photographic reagents, pharmaceutical intermediates, electroplating and paint-spraying, and can be upgraded through chemical means to prepare a variety of chemicals and fuels.

There are many synthesis processes for 2,5-hexanedione. A traditional process is a synthesis of starting from ethyl acetoacetate under the action of $Na/Et_2O$, then being coupled with $I_2$, and then decarboxylation under an alkaline condition. However, this process is costly and unsafe to operate, leading to its high price. Biomass is the only renewable organic carbon source, so starting from biomass has become a hotspot in current studies. For example, the platform compound 5-hydroxymethylfurfural prepared from biomass is hydrolyzed and hydrogenated to prepare 2,5-hexanedione (Green Chemistry. 2016, 18, 3075-3081; Green Chemistry. 2016, 18, 2956-2960; ChemSusChem 2014, 7, 96-100; CN105693486A), and 2,5-dimethylfuran is hydrolyzed to prepare 2,5-hexanedione (CN105348056A; CN101423467B) and the like. However, the raw materials 5-hydroxy methyl furfural and 2,5-dimethylfuran used in the above preparation processes are expensive, resulting in high costs and low economic benefits for the preparation of 2,5-hexanedione.

Jérôme research group (ChemSusChem 2014, 7, 96-100) reports catalytically preparing 2,5-hexanedione from fructose in one step using Pd/C as the hydrogenation catalyst and high-pressure $CO_2$ as the acid catalyst, but the yield of 2,5-hexanedione is only 28%, and the raw materials are limited to fructose. Subsequently, Essayem research group (Applied Catalysis A: General, 2015, 504, 664-671) reports the preparation of 2,5-hexanedione from cellulose as raw material with ZrW as the catalyst, wherein the highest yield of 2,5-hexanedione is only 24.5%, and the yield is relatively low. CN109896938A discloses using virgin biomass as raw material and using liquid acid and supported noble metal as catalyst so that the yield of 2,5-hexanedione can reach 65%. However, the liquid acids are used as the catalyst in the above reactions, which will cause a certain degree of equipment corrosion, and the used liquid acid will cause environmental pollution and high treatment costs, which causes major problems in the practical industrial applications. Therefore, an efficient and green process is needed to achieve efficient one-pot catalytic conversion of biomass to prepare 2,5-hexanedione.

**Summary of the Invention**

The technical problems to be solved by the present invention are the problems existing in the prior art such as low catalytic efficiency or environmental pollution caused by liquid acid, and therefore the present invention provides a one-pot process for catalytically converting biomass to prepare 2,5-hexanedione. The process can achieve efficient conversion of biomass without the participation of acid catalysts, and have a very high selectivity for the product 2,5-hexanedione.

In order to solve the above technical problems, the present invention provides a biphasic solvent system for converting biomass to prepare 2,5-hexanedione, which contains an organic solvent phase and an aqueous solution phase, wherein: the aqueous solution phase contains an anion selected from elements of Group VIIA; the aqueous solution phase has a pH ranging from about 6.5 to about 8.5, and preferably from 7 to 8; and contains a hydrophobic hydrogenation catalyst for preparing 2,5-hexanedione from biomass.

The organic solvent phase and the aqueous solution phase form a biphasic solvent system. As an example, in an embodiment, the organic solvent phase can have a lower density than that of the aqueous solution phase, and range from about 0.8 to about 0.95 $Kg/m^3$.

In an embodiment, the aqueous solution phase further contains a cation from elements of Group IA that is in an equimolar amount with the anion from elements of Group VIIA and can form an inorganic salt with the anion from elements of Group VIIA.

The elements of Group VIIA are halogen elements, and the elements of Group IA are alkali metal elements; accordingly, the inorganic salts formed from their anions and cations are typically neutral and can exhibit a pH of about 7.

In an embodiment, the inorganic salt is a chloride or a bromide. For example, the inorganic salt may be LiCl, NaCl, KCl, LiBr, NaBr or KBr.

In this field, it is usual to add a liquid acid or an acidic salt into the reaction system in the existing one-step process of catalytically converting biomass to 2,5-hexanedione so as to play a catalytic role together with a supported noble metal. That is to say, in known conventional processes, an acidic reaction environment is usually maintained. Without being limited to any known theory, the inventors have found through in-depth studies that introducing a halogen anion into the reaction system and maintaining it at a certain concentration, and allowing the reaction to start from a roughly neutral pH can exhibit an excellent reactivity together with a supported noble metal.

A one-pot process for catalytically converting biomass to prepare 2,5-hexanedione comprises: contacting and reacting a biomass raw material with a hydrogenation catalyst using hydrogen gas as a hydrogen source in a heterogeneous system formed from an organic solvent, an inorganic salt and water to obtain 2,5-hexanedione; the hydrogenation catalyst comprises a hydrogenation active component and a support, wherein said support is selected from one or more of hydrophobic active carbon and graphene.

According to the present invention, said organic solvent is selected from tetrahydrofuran, toluene, methyl isobutyl ketone, 1,4-dioxane, $\gamma$-valerolactone, chloroform, 1,2-dichloroethane, and mixtures thereof.

According to the present invention, the anion and the cation in the inorganic salt are respectively from elements of Group VIIA and elements of Group IA, wherein the element of Group VIIA is selected from at least one of Cl and Br, and the element of Group IA is selected from at least one of Li, Na, and K.

According to the present invention, the ratio of the weight of the organic solvent to the total weight of the inorganic salt and water ranges from 2 to 16, and preferably from 3 to 10; and/or, the ratio of the weight of the inorganic salt to the weight of water ranges from 0.10 to 0.70, for example, it can be but is not limited to 0.10, 0.20, 0.30, 0.40, 0.50, 0.60, 0.70 and a range between any two, preferably from 0.20 to 0.70, and further preferably from 0.40 to 0.70. In the present invention, when the weight ratio of the inorganic salt to the water reaches 0.40 or higher, and in the presence of the hydrophobic catalyst of the present invention, there is a more prominent effect in improving the selectivity for the product 2,5-hexanedione.

According to the present invention, the weight ratio of the organic solvent to the biomass raw material ranges from 5 to 60, and preferably from 15 to 40.

According to the present invention, the hydrogenation active component is selected from one or more of ruthenium, platinum, and palladium, and preferably platinum and/or palladium. Based on the weight of the hydrogenation catalyst on a dry basis, in terms of atoms, the hydrogenation active component is present in an amount ranging from 0.5% to 10%, and preferably from 2% to 6%.

According to the present invention, based on the weight of the hydrogenation catalyst on a dry basis, the support is present in an amount ranging from 90% to 99.5%, and preferably from 94% to 98%.

According to the present invention, the contact angle between the hydrogenation catalyst and water is greater than 50°, preferably ranges from 55° to 90°, and still preferably from 60° to 90°, examples thereof include, but are not limited thereto: 55°, 60°, 65°, 70°, 75°, 80°, 85°, and 90°.

According to the present invention, the biomass raw material is one or more of cellulose, glucose, fructose, sucrose, inulin, starch, corn straw, corn cob, sugar cane bagasse, and the like.

According to the present invention, in the reaction system, the hydrogen pressure ranges from 0.2 MPa to 6 MPa, and preferably from 0.5 MPa to 3 MPa.

According to the present invention, the weight ratio of the biomass raw material to the hydrogenation catalyst is (8-0.5): 1, and preferably (4-1): 1; and/or, the reaction temperature ranges from 160°C to 240°C, and preferably from 180°C to 220°C; and/or, the reaction time ranges from 2 hours to 16 hours, and preferably from 4 hours to 12 hours.

According to the present invention, the support can be a hydrophobic support, which is prepared by using a high-temperature calcining process, the process specifically comprising: calcining active carbon and/or graphene at a high temperature with an inert gas as carrier gas to produce a hydrophobic support. In the process, the conditions for high-temperature calcining are as follows: the calcining temperature ranges from 400°C to 900°C, and the calcining time ranges from 3 hours to 12 hours.

According to the present invention, the hydrogenation catalyst can be prepared by an impregnation process (preferably an isovolumetric impregnation process), which specifically comprises: impregnating an aqueous solution containing a hydrogenation active metal on the support, followed by drying, calcining and reducing to produce a hydrogenation catalyst, wherein the solution containing the hydrogenation active metal can be formulated with soluble metal compounds, such as nitrate, chloride, acetate, and chloroplatinic acid. The present invention has no particular limitation on the impregnation conditions. For example, the impregnation can be performed at room temperature for 1-10 hours. The drying can be performed in a conventional manner, preferably, the drying temperature ranges from 40°C to 90°C, and the drying time ranges from 4 hours to 12 hours. The calcining can be performed in a conventional manner, preferably, the calcining temperature ranges from 300°C to 550°C, and the calcining time ranges from 3 hours to 8 hours. The reducing can be performed with hydrogen gas, and the reducing conditions are preferably as follows: the reducing temperature ranges from 300°C to 450°C, and the reducing time ranges from 3 hours to 6 hours.

According to the present invention, the reaction product is centrifugally separated to produce an organic phase containing 2,5-hexanedione, which mainly contains 2,5-hexanedione and an organic solvent. Subsequently, conventional processes, such as rectification, can be used for separation to produce 2,5-hexanedione.

The present invention therefore provides the following exemplary embodiments:

1. A one-pot process for catalytically converting biomass to prepare 2,5-hexanedione, which comprises: contacting and reacting a biomass raw material with a hydrogenation catalyst using hydrogen gas as a hydrogen source in a heterogeneous system formed from an organic solvent, an inorganic salt and water to obtain 2,5-hexanedione; the hydrogenation catalyst comprises a hydrogenation active component and a support, wherein said support is selected from one or more of hydrophobic active carbon and graphene.

2. The process according to exemplary embodiment 1, characterized in that said organic solvent is selected from tetrahydrofuran, toluene, methyl isobutyl ketone, 1,4-dioxane, γ-valerolactone, chloroform, 1,2-dichloroethane, and mixtures thereof.

3. The process according to exemplary embodiment 1 or 2, characterized in that the anion and the cation in the inorganic salt are respectively from elements of Group VIIA and elements of Group IA, wherein the element of Group VIIA is selected from at least one of Cl and Br, and the element of Group IA is selected from at least one of Li, Na, and K.

4. The process according to exemplary embodiment 3, characterized in that, the ratio of the weight of the organic solvent to the total weight of the inorganic salt and water ranges from 2 to 16, and preferably from 3 to 10; and/or, the weight ratio of the inorganic salt to the water ranges from 0.10 to 0.70, preferably from 0.20 to 0.70, and further preferably from 0.40 to 0.70.

5. The process according to any one of exemplary embodiments 1-4, characterized in that, the weight ratio of the organic solvent to the biomass raw material ranges from 5 to 60, and preferably from 15 to 40.

6. The process according to exemplary embodiment 1, characterized in that the hydrogenation active component is selected from one or more of ruthenium, platinum, and palladium, and preferably platinum and/or palladium; preferably, based on the weight of the hydrogenation catalyst, in terms of atoms, the hydrogenation active component is present in an amount ranging from 0.5% to 10%, and preferably from 2% to 6%.

7. The process according to exemplary embodiment 1 or 6, characterized in that the contact angle between the hydrogenation catalyst and water is greater than 50°, and preferably ranges from 55° to 90°.

8. The process according to exemplary embodiment 1, characterized in that the biomass raw material is one or more of cellulose, glucose, fructose, sucrose, inulin, starch, corn straw, corn cob, and sugar cane bagasse.

9. The process according to exemplary embodiment 1, characterized in that in the reaction system, the hydrogen pressure ranges from 0.2 MPa to 6 MPa, and preferably 0.5 MPa to 3 MPa.

10. The process according to any one of exemplary embodiments 1-9, characterized in that the weight ratio of the biomass raw material to the hydrogenation catalyst is (8-0.5): 1, and preferably (4-1): 1; and/or, the reaction temperature ranges from 160°C to 240°C, and preferably from 180°C to 220°C; and/or, the reaction time ranges from 2 hours to 16 hours, and preferably from 4 hours to 12 hours.

Compared with the prior art, the beneficial effects of the present invention comprise:
The present invention uses virgin biomass as raw material, which is cheap and widely sourced. No acid catalyst is used in the reaction process, which avoids problems such as corrosion of equipment, environmental pollution, and high processing costs caused by acid. The process is simple and can efficiently convert the biomass. The prepared 2,5-hexanedione product has very high selectivity, the reaction system has good cycle stability, and has good industrial application prospects.

## Description of the drawings

Figure 1 is a figure for the measurement result of the contact angle between the hydrogenation catalyst obtained in Example 1 and water;

Figure 2 is a figure for the measurement result of the contact angle between the hydrogenation catalyst obtained in Comparative Example 1 and water.

**Detailed description**

Herein unless otherwise stated, all technical features and preferred features mentioned herein regarding various aspects, various series and/or various embodiments can be combined with each other to form new technical solutions.

Herein unless otherwise stated, specific steps, specific values, and specific materials described in examples can be combined with other features in other parts of the specification. For example, if it is mentioned in "Summary of the Invention" or "Detailed description" of the specification that the reaction temperature ranges from 10°C to 100°C, and the specific reaction temperature disclosed in examples is 20°C, then it may be considered that the range from 10°C to 20°C or the range from 20°C to 100°C is specifically disclosed herein, and this range can be combined with other features in other parts of the specification to form new technical solutions.

Herein unless otherwise stated, the terms such as "include", "comprise", "contain", and "have" are in an open-ended manner, but it should also be understood that these terms also disclose a scenario in a close-ended manner. For example, "including" indicates that other elements not listed may also be included, but it also explicitly discloses that only the listed elements are included.

Herein unless otherwise stated, specific steps, specific values, and specific materials described in examples can be combined with other features in other parts of the specification. For example, if it is mentioned in "Summary of the Invention" or "Detailed description" of the specification that the reaction temperature ranges from 10°C to 100°C, and the specific reaction temperature disclosed in examples is 20°C, then it may be considered that the range from 10°C to 20°C or the range from 20°C to 100°C is specifically disclosed herein, and this range can be combined with other features in other parts of the specification to form new technical solutions.

In the present invention, the reaction product 2,5-hexanedione (HDO) is analyzed qualitatively by gas chromatography-mass spectrometry (GC-MS), and the yield of the product 2,5-hexanedione is analyzed by gas chromatography (GC). The gas chromatograph-mass spectrometer instrument is Agilent 7890A from Agilent Company of the United States, the chromatographic column is HP-INNOWax capillary column (30m, 0.53mm), the gas chromatograph is Agilent 7890B, the detector is a hydrogen flame ion detector (FID), and the chromatographic column is HP-INNOWax capillary column (30m, 0.53mm).

In the present invention, the calculation formula for the yield of the product 2,5-hexanedione is:

$$\text{The yield \% of the product 2,5-hexanedione} = (\text{the molar amount of 2,5-hexanedione produced in the reaction}) / (\text{the molar amount of hexose units in the reactants}) \times 100\%,$$

wherein the hexose unit is $C_6H_{10}O_5$.

In the present invention, the contact angle is measured with a measuring instrument model DSA100 from KRUSS Company of Germany. A tangent line to the gas-liquid interface is plotted from the intersection of gas, liquid and solid phases. The angle $\theta$ between the tangent and the solid-liquid boundary passing through the three-phase contact point is the contact angle of the liquid on the solid surface. If the gas is air, the solid is the hydrogenation catalyst, and the liquid is water, the measured contact angle is the contact angle between hydrogenation catalyst and water. The larger the contact angle, the better the relative hydrophobicity of the hydrogenation catalyst.

In order to facilitate the understanding of the present invention, the following examples are enumerated, but these examples are only used to assist in understanding the present invention and should not be considered as specific limitations to the present invention.

**Example 1**

First, 5g graphene sample was treated in a 90°C oven for 4 hours, and then transferred to a high-temperature tube furnace. Nitrogen gas was introduced as carrier gas, and the gas volumetric space velocity was 2 h$^{-1}$. The temperature was increased to 750°C at a ramp rate of 5°C, and kept for 8 hours to produce a hydrophobic graphene (expressed as Gr).

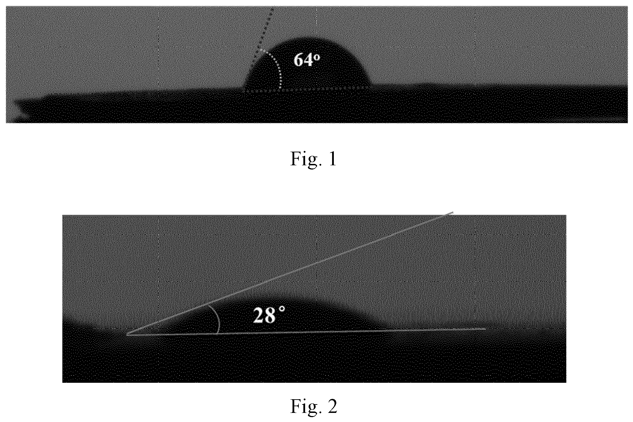

Preparation of catalyst 3% Pd/Gr: Palladium nitrate was impregnated on the above-mentioned hydrophobic graphene by means of an isovolumetric impregnation method. The impregnation amount was calculated according to the weight ratio of noble metal Pd:Gr of 3:100. The impregnated graphene was treated in a 90°C oven for 8 hours, and then transferred to a high-temperature tube furnace. Nitrogen gas was introduced as carrier gas, and the gas volumetric space velocity was 2 h$^{-1}$. The temperature was increased to 500°C at a ramp rate of 10°C, kept for 4 hours, and then decreased to room temperature to produce PdO/Gr. The carrier gas was switched to hydrogen gas, and the gas volumetric space velocity was 2 h$^{-1}$. The temperature was increased to 400°C at a ramp rate of 10°C, and kept for 4 hours. Then the carrier gas was switched to nitrogen gas again, and the temperature was decreased to room temperature to produce 3% Pd/Gr. It was found through the subsequent measurement that the contact angle between the catalyst and water was 64°, as shown in Figure 1, indicating that the material had relatively good hydrophobicity.

**Example 2**

First, 5g active carbon sample was treated in a 90°C oven for 4 hours, and then transferred to a high-temperature tube furnace. Nitrogen gas was introduced as carrier gas, and the gas volumetric space velocity was $2\,h^{-1}$. The temperature was increased to 700°C at a ramp rate of 5°C, and kept for 8 hours to produce a hydrophobic active carbon (expressed as C).

Preparation of catalyst 3% Pd/C: Palladium nitrate was impregnated on the above-mentioned hydrophobic active carbon by means of an isovolumetric impregnation method. The impregnation amount was calculated according to the weight ratio of noble metal Pd:C of 3:100. The impregnated active carbon was treated in a 80°C oven for 6 hours, and then transferred to a high-temperature tube furnace. Nitrogen gas was introduced as carrier gas, and the gas volumetric space velocity was $2\,h^{-1}$. The temperature was increased to 450°C at a ramp rate of 10°C, kept for 4 hours, and then decreased to room temperature to produce PdO/C. The carrier gas was switched to hydrogen gas, and the gas volumetric space velocity was $2\,h^{-1}$. The temperature was increased to 400°C at a ramp rate of 10°C, and kept for 4 hours. Then the carrier gas was switched to nitrogen gas again, and the temperature was decreased to room temperature to produce 3% Pd/C. It was found through the subsequent measurement that the contact angle between the catalyst and water was 57°, which was similar to that in Figure 1, indicating that the material had relatively good hydrophobicity.

**Example 3**

First, 5g graphene sample was treated in a 90°C oven for 4 hours, and then transferred to a high-temperature tube furnace. Helium gas was introduced as carrier gas, and the gas volumetric space velocity was $2\,h^{-1}$. The temperature was increased to 800°C at a ramp rate of 5°C, and kept for 8 hours to produce a hydrophobic graphene.

Preparation of catalyst 5% Pt/Gr: Chloroplatinic acid was impregnated on the above-mentioned hydrophobic graphene by means of an isovolumetric impregnation method. The impregnation amount was calculated according to the weight ratio of noble metal Pt:Gr of 5:100. The impregnated graphene was treated in a 70°C oven for 8 hours, and then transferred to a high-temperature tube furnace. Nitrogen gas was introduced as carrier gas, and the gas volumetric space velocity was $2\,h^{-1}$. The temperature was increased to 500°C at a ramp rate of 10°C, kept for 4 hours, and then decreased to room temperature to produce PtO/Gr. The carrier gas was switched to hydrogen gas, and the gas volumetric space velocity was $2\,h^{-1}$. The temperature was increased to 350°C at a ramp rate of 10°C, and kept for 5 hours. Then the carrier gas was switched to nitrogen gas again, and the temperature was decreased to room temperature to produce 5% Pt/Gr. It was found through the subsequent measurement that the contact angle was 76°, which was similar to that in Figure 1, indicating that the material had relatively good hydrophobicity.

**Example 4**

Glucose was used as biomass raw material. The weight ratio of glucose to the 3% Pd/Gr catalyst in Example 1 was 2:1, the weight ratio of the organic solvent to glucose was 20:1, the weight ratio of the organic solvent to NaCl and water was 8, and the weight ratio of NaCl to water was 0.50. To a high-pressure magnetic stirring batch reactor were respectively added 0.5g of the catalyst 3% Pd/Gr in Example 1, 2.5g of NaCl and water (the weight ratio of NaCl to water was 0.50), 1.0g of glucose, and 20g of tetrahydrofuran as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 62%.

**Example 5**

Glucose was used as biomass raw material. The weight ratio of glucose to the 3% Pd/C catalyst in Example 2 was 2:1, the weight ratio of the organic solvent to glucose was 15:1, the weight ratio of the organic solvent to NaCl and water was 6, and the weight ratio of NaCl to water was 0.42. To a high-pressure magnetic stirring batch reactor were respectively added 0.5g of the catalyst 3% Pd/C in Example 2, 2.5g of NaCl and water (the weight ratio of NaCl to water was 0.42), 1.0g of glucose, and 15g of tetrahydrofuran as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 58%.

**Example 6**

Glucose was used as biomass raw material. The weight ratio of glucose to the 5% Pt/Gr catalyst in Example 3 was 2:1, the weight ratio of the organic solvent to glucose was 20:1, the weight ratio of the organic solvent to NaCl and water was 8, and the weight ratio of NaCl to water was 0.25. To a high-pressure magnetic stirring batch reactor were respectively added 0.5g of the catalyst 5% Pt/Gr in Example 3, 2.5g of NaCl and water (the weight ratio of NaCl to water was 0.25), 1.0g of glucose, and 20g of toluene as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 52%.

**Example 7**

Glucose was used as biomass raw material. The weight ratio of glucose to the 3% Pd/C catalyst in Example 2 was 2:1, the weight ratio of the organic solvent to glucose was 35:1, the weight ratio of the organic solvent to NaCl and water was 5, and the weight ratio of NaCl to water was 0.28. To a high-pressure magnetic stirring batch reactor were respectively added 0.5g of the catalyst 3% Pd/C in Example 2, 7.0g of NaCl and water (the weight ratio of NaCl to water was 0.28), 1.0g of glucose, and 35g of toluene as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 48%.

**Example 8**

Glucose was used as biomass raw material. The weight ratio of glucose to the 5% Pt/Gr catalyst in Example 3 was 2:1, the weight ratio of the organic solvent to glucose was 40:1, the weight ratio of the organic solvent to NaCl and water was 7, and the weight ratio of NaCl to water was 0.26. To a high-pressure magnetic stirring batch reactor were respectively added 0.5g of the 5% Pt/Gr catalyst in Example 3, 5.7g of NaCl and water (the weight ratio of NaCl to water was 0.26), 1.0g of glucose, and 40g of methyl isobutyl ketone as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 55%.

**Example 9**

Glucose was used as biomass raw material. The weight ratio of glucose to the 3% Pd/Gr catalyst in Example 1 was 2:1, the weight ratio of the organic solvent to glucose was 18:1, the weight ratio of the organic solvent to KCl and water was 4, and the weight ratio of KCl to water was 0.55. To a high-pressure magnetic stirring batch reactor were respectively added 0.5g of the 3% Pd/Gr catalyst in Example 1, 4.5g of KCl and water (the weight ratio of KCl to water was 0.55), 1.0g of glucose, 18 g of methyl isobutyl ketone as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 62%.

**Example 10**

Glucose was used as biomass raw material. The weight ratio of glucose to the 5% Pt/Gr catalyst in Example 3 was 2:1, the weight ratio of the organic solvent to glucose was 18:1, the weight ratio of the organic solvent to KBr and water was 8, and the weight ratio of KBr to water was 0.24. To a high-pressure magnetic stirring batch reactor were respectively added 0.5g of the 5% Pt/Gr catalyst in Example 3, 2.3 g of KBr and water (the weight ratio of KBr to water was 0.24), 1.0g of glucose, and 18 g of 1,4-dioxane as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 54%.

**Example 11**

Glucose was used as biomass raw material. The weight ratio of glucose to the 3% Pd/Gr catalyst in Example 1 was 2:1, the weight ratio of the organic solvent to glucose was 25:1, the weight ratio of the organic solvent to NaCl and water was 5, and the weight ratio of NaCl to water was 0.20. To a high-pressure magnetic stirring batch reactor were respectively added 0.5g of the 3% Pd/Gr catalyst in Example 1, 5.0 g of NaCl and water (the weight ratio of NaCl to water was 0.20), 1.0g of glucose, and 25g of 1,4-dioxane as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 48%.

**Example 12**

Glucose was used as biomass raw material. The weight ratio of glucose to the 3% Pd/C catalyst in Example 2 was 2:1, the weight ratio of the organic solvent to glucose was 25:1, the weight ratio of the organic solvent to a concentrated brine of NaCl and water was 8, and the weight ratio of NaCl to water was 0.25. To a high-pressure magnetic stirring batch reactor were respectively added 0.5g of the catalyst 3% Pd/C in Example 2, 3.1 g of NaCl and water (the weight ratio of NaCl to water was 0.25), 1.0g of glucose, and 25g of $\gamma$-valerolactone as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 49%.

**Example 13**

Glucose was used as biomass raw material. The weight ratio of glucose to the 5% Pt/Gr catalyst in Example 3 was 2:1, the weight ratio of the organic solvent to glucose was 20:1, the weight ratio of the organic solvent to NaCl and water was 8, and the weight ratio of NaCl to water was 0.28. To a high-pressure magnetic stirring batch reactor were respectively added 0.5g of the 5% Pt/Gr catalyst in Example 3, 2.5 g of NaCl and water (the weight ratio of NaCl to water was 0.28), 1.0g of glucose, and 20g of chloroform as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 53%.

**Example 14**

Glucose was used as biomass raw material. The weight ratio of glucose to the 3% Pd/C catalyst in Example 2 was 2:1, the weight ratio of the organic solvent to glucose was 20:1, the weight ratio of the organic solvent to NaCl and water was 8, and the weight ratio of NaCl to water was 0.30. To a high-pressure magnetic stirring batch reactor were respectively added 0.5g of the catalyst 3% Pd/C in Example 2, 2.5 g of NaCl and water (the weight ratio of NaCl to water was 0.30), 1.0g of glucose, and 20g of 1,2-dichloroethane as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 52%.

**Example 15**

Glucose was used as biomass raw material. The weight ratio of glucose to the 3% Pd/C catalyst in Example 2 was 2:1, the weight ratio of the organic solvent to glucose was 20:1, the weight ratio of the organic solvent to NaCl and water was 8, and the weight ratio of NaCl to water was 0.55. To a high-pressure magnetic stirring batch reactor were respectively added 0.5g of the catalyst 3% Pd/C in Example 2, 2.5 g of NaCl and water (the weight ratio of NaCl to water was 0.55), 1.0g of glucose, and 20g of 1,2-dichloroethane as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 61%.

In order to describe the reaction conditions and results of the above-mentioned Examples 4-15 more intuitively, various parameters and results are listed in Table 1.

Table 1: Reaction conditions and reaction results of Examples 4-15

| Example | Hydrogenation catalyst | Organic solvent | Mass ratio of organic solvent to (inorganic salt and water) | Mass ratio of organic solvent to raw material glucose | Mass ratio of inorganic salt to water | Conversion of glucose/% | Yield of 2,5-hexanedione/% |
|---|---|---|---|---|---|---|---|
| 4 | Example 1 | tetrahydrofuran | 8 | 20 | 0.50 | >99 | 62 |
| 5 | Example 2 | tetrahydrofuran | 6 | 15 | 0.42 | >99 | 58 |
| 6 | Example 3 | toluene | 8 | 20 | 0.25 | >99 | 52 |
| 7 | Example 2 | toluene | 5 | 35 | 0.28 | >99 | 48 |
| 8 | Example 3 | methyl isobutyl ketone | 7 | 40 | 0.26 | >99 | 55 |
| 9 | Example 1 | methyl isobutyl ketone | 4 | 18 | 0.55 | >99 | 62 |
| 10 | Example 3 | 1,4-dioxane | 8 | 18 | 0.24 | >99 | 54 |
| 11 | Example 1 | 1,4-dioxane | 5 | 25 | 0.20 | >99 | 48 |
| 12 | Example 2 | $\gamma$-valerolactone | 8 | 25 | 0.25 | >99 | 49 |
| 13 | Example 3 | chloroform | 8 | 20 | 0.28 | >99 | 53 |
| 14 | Example 2 | 1,2-dichloroethane | 8 | 20 | 0.30 | >99 | 52 |
| 15 | Example 2 | 1,2-dichloroethane | 8 | 20 | 0.55 | >99 | 61 |

**Examples 16-24**

To a high-pressure magnetic stirring batch reactor were respectively added the catalyst 3% Pd/Gr in Example 1, 4.0g of NaCl and water (the weight ratio of NaCl to water was 0.30), 1.0g of glucose, 30g of tetrahydrofuran as organic solvent. Hydrogen gas at a certain pressure was introduced, and the reaction system was heated to a certain temperature and kept at this temperature for a certain period. After the completion of the reaction, the reaction system was cooled to room temperature, and centrifugally separated to obtain an organic phase containing 2,5-hexanedione, which was subjected to the gas chromatography analysis. The yield of 2,5-hexanedione was obtained by calculation, and the results were shown in Table 2.

Table 2: Preparation of 2,5-hexanedione under different reaction conditions

| Example | Mass ratio of raw material to catalyst | Hydrogen pressure (MPa) | Reaction temperature (°C) | Reaction time (h) | Conversion of glucose/% | Yield of 2,5-hexanedione/% |
|---|---|---|---|---|---|---|
| 16 | 1 | 2 | 200 | 6 | >99 | 54 |
| 17 | 1 | 3 | 220 | 8 | >99 | 55 |
| 18 | 2 | 1.5 | 180 | 12 | >99 | 54 |
| 19 | 2 | 1 | 190 | 10 | >99 | 49 |
| 20 | 2 | 1.5 | 220 | 6 | >99 | 46 |
| 21 | 0.5 | 1 | 200 | 9 | >99 | 52 |
| 22 | 0.5 | 1.5 | 210 | 7 | >99 | 51 |
| 23 | 1.5 | 1.5 | 200 | 12 | >99 | 56 |
| 24 | 1.5 | 2 | 190 | 10 | >99 | 55 |

**Examples 25-32**

To a high-pressure magnetic stirring batch reactor were respectively added 0.5g of the catalyst 3% Pd/C in Example 2, 4.0g of NaCl and water (the weight ratio of NaCl to water was 0.30), 0.5g of a different raw material, 20g of tetrahydrofuran as organic solvent. Hydrogen gas was introduced until the hydrogen pressure was 1.5 MPa, and the reaction system was heated to 200°C and kept at this temperature for 8 hours. After the completion of the reaction, the reaction system was cooled to room temperature, and centrifugally separated to obtain an organic phase containing 2,5-hexanedione, which was subjected to the gas chromatography analysis. The yield of 2,5-hexanedione was obtained by calculation, and the results were shown in Table 3.

Table 3: Preparation of 2,5-hexanedione with a different biomass as raw material

| Example | Raw material | Yield of 2,5-hexanedione/% |
|---|---|---|
| 25 | cellulose | 52 |
| 26 | fructose | 52 |
| 27 | sucrose | 54 |
| 28 | inulin | 52 |
| 29 | starch | 53 |
| 30 | corn straw | 60 |
| 31 | corn cob | 58 |
| 32 | sugar cane bagasse | 54 |

**Example 33**

A cycle stability test was performed, and the operation procedure was as follows. The tetrahydrofuran solvent organic phase material in the upper layer of the reaction solution in Example 4 was directly separated and the yield of 2,5-hexanedione was analyzed. The rest materials in the lower layer were retained. Then the reaction substrates of 1.0 g of glucose and 20g of tetrahydrofuran solvent were fed into the reactor to take part in the new reaction. Hydrogen gas was introduced until the hydrogen pressure was 2 MPa, and the reaction system was heated to 200°C and kept at this temperature for 8 hours. Then, the reaction system was cooled to room temperature, and centrifugally separated to obtain an organic phase containing 2,5-hexanedione, which was subjected to the gas chromatography analysis. The yield of 2,5-hexanedione was obtained by calculation, and the cycle results were shown in Table 4. The results showed that the yield of 2,5-hexanedione remained almost unchanged when the cycle operation reached the fifth cycle, indicating that the reaction system had good cycle stability.

Table 4: Results for cycle use

| Cycle number | Conversion of glucose/% | Yield of 2,5-hexanedione/% |
|---|---|---|
| 1 | >99 | 62 |
| 2 | >99 | 60 |
| 3 | >99 | 60 |
| 4 | >99 | 59 |
| 5 | >99 | 58 |

**Comparative Example 1**

This example was carried out with reference to Example 12 except preparation of catalyst 3% Pd/DC: Palladium nitrate was impregnated on the untreated active carbon (expressed as DC) of Example 2 by means of an isovolumetric impregnation method. The impregnation amount was calculated according to the weight ratio of noble metal Pd:DC of 3:100. The impregnated active carbon was treated in an 80°C oven for 6 hours, and then transferred to a high-temperature tube furnace. Nitrogen gas was introduced as carrier gas, and the gas volumetric space velocity was 2 h$^{-1}$. The temperature was increased to 450°C at a ramp rate of 10°C, kept for 4 hours, and then decreased to room temperature. The carrier gas was switched to hydrogen gas, and the gas volumetric space velocity was 2 h$^{-1}$. The temperature was increased to 400°C at a ramp rate of 10°C, and kept for 4 hours. Then the carrier gas was switched to nitrogen gas again, and the temperature was decreased to room temperature to produce 3% Pd/DC. It was found through the subsequent measurement that the contact angle was about 28°, as shown in Figure 2, indicating that the material had relatively poor hydrophobicity.

Glucose was used as biomass raw material. The weight ratio of glucose to the 3% Pd/DC catalyst in Comparative Example 1 was 2:1, the weight ratio of the organic solvent to glucose was 25: 1, the weight ratio of the organic solvent to NaCl and water was 8, and the weight ratio of NaCl to water was 0.25. To a high-pressure magnetic stirring batch reactor were respectively added 0.5g of the catalyst 3% Pd/DC in Comparative Example 1, 3.1 g of NaCl and water (the weight ratio of NaCl to water was 0.25), 1.0g of glucose, 25 g of γ-valerolactone as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 25%.

**Comparative Example 2**

Glucose was used as biomass raw material. The weight ratio of glucose to the 3% Pd/Gr catalyst in Example 1 was 2:1, the weight ratio of the organic solvent to glucose was 20:1, the water phase was deionized water, and the weight ratio of the organic solvent to deionized water was 8. To a high-pressure magnetic stirring batch reactor were respectively added 0.5g of the catalyst 3% Pd/Gr in Example 1, 2.5 g of deionized water, 1.0g of glucose, 20g of tetrahydrofuran as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was 68%, and the yield of 2,5-hexanedione was 5%.

**Comparative Example 3**

This example was carried out with reference to Example 4 except that the weight ratio of glucose to the 3% Pd/Gr catalyst in Example 1 was 2:1, the weight ratio of the organic solvent to glucose was 20:1, the weight ratio of organic solvent to $Na_2SO_4$ and water was 8, and the weight ratio of $Na_2SO_4$ to water was 0.50. To a high-pressure magnetic stirring batch reactor were respectively added 0.5g of the catalyst 3% Pd/Gr in Example 1, 2.5g of $Na_2SO_4$ and water (the weight ratio of $Na_2SO_4$ to water was 0.50), 1.0g of glucose, and 20g of tetrahydrofuran as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 4%.

**Comparative Example 4**

This example was carried out with reference to Example 4 except that the weight ratio of glucose to the 3% Pd/Gr catalyst in Example 1 was 2:1, the weight ratio of the organic solvent to glucose was 20:1, the weight ratio of organic solvent to CaCh and water was 8, and the weight ratio of CaCh to water was 0.50. To a high-pressure magnetic stirring batch reactor were respectively added 0.5g of the catalyst 3% Pd/Gr in Example 1, 2.5g of CaCh and water (the weight ratio of CaCh to water was 0.50), 1.0g of glucose, and 20g of tetrahydrofuran as organic solvent. Hydrogen gas was introduced until the hydrogen pressure was 2MPa, and the reaction system was heated to 200°C and kept at this temperature for 8 hours before cooling to room temperature, and then centrifugally separated to obtain an organic phase containing 2,5-hexanedione, which was subjected to the gas chromatography analysis. By calculation, the conversion of glucose was >99%, and the yield of 2,5-hexanedione was 27%.

The specific embodiments of the present invention have been described in detail above, but the present invention is not limited thereto. Within the scope of the technical concept of the present invention, many simple modifications can be made to the technical solution of the present invention, including the combination of various technical features in any other suitable manner. These simple modifications and combinations should also be regarded as the disclosed content of the present invention, and all belong to the protection scope of the present invention.

**Claims**

1.  A biphasic solvent system for converting biomass to prepare 2,5-hexanedione, comprising an organic solvent phase and an aqueous solution phase,

    wherein the aqueous solution phase contains an anion selected from elements of Group VIIA; the pH of the aqueous solution phase ranges from about 6.5 to about 8.5, and preferably from 7 to 8 under the room temperature condition of 25°C, and
    the organic solvent phase contains a hydrophobic hydrogenation catalyst for the preparation of 2,5-hexanedione from biomass.

2.  The system according to claim 1, which is **characterized in that** the aqueous solution phase further contains a cation from elements of Group IA that is in an equimolar amount with the anion from elements of Group VIIA and can form an inorganic salt with the anion from elements of Group VIIA.

3.  The system according to claim 2, which is **characterized in that** the element of Group VIIA is selected from at least one of Cl and Br, and/or the element of Group IA is selected from at least one of Li, Na, and K.

4.  The system according to claim 1, which is **characterized in that** the hydrophobic hydrogenation catalyst comprises a hydrogenation active component and a support, wherein said support is selected from one or more of hydrophobic active carbon and graphene.

5.  The system according to any one of claims 1-4, which is **characterized in that** the organic solvent of the organic solvent phase is selected from tetrahydrofuran, toluene, methyl isobutyl ketone, 1,4-dioxane, γ-valerolactone, chloroform, 1,2-dichloroethane, and mixtures thereof.

6.  The system according to claim 5, which is **characterized in that** the anion from elements of Group VIIA and the cation from elements of Group IA are added by adding an inorganic salt containing the anion and the cation; wherein the ratio of the weight of the organic solvent in the organic solvent phase to the total weight of the inorganic salt and water in the aqueous solution phase ranges from 2 to 16, and preferably from 3 to 10; and/or, the weight ratio of the inorganic salt to the water ranges from 0.10 to 0.70, preferably from 0.20 to 0.70, and further preferably from 0.40 to 0.70.

7. The system according to claim 1, which is **characterized in that** the hydrogenation active component is selected from one or more of ruthenium, platinum, and palladium, and preferably platinum and/or palladium; preferably, based on the weight of the hydrogenation catalyst on a dry basis, in terms of atoms, the hydrogenation active component is present in an amount ranging from 0.5% to 10% by weight, and preferably from 2% to 6% by weight.

8. The system according to claim 1 or 7, which is **characterized in that** the contact angle between the hydrogenation catalyst and water is greater than 50°, preferably ranges from 55° to 90°, and still preferably ranges from 60° to 90°.

9. The system according to any one of claims 1-4, which is **characterized in that** the organic solvent phase has a lower density than that of the aqueous solution phase, for example the organic solvent phase has a density ranging from about 0.8 Kg/m$^3$ to about 0.95 Kg/m$^3$.

10. A one-pot process for catalytically converting biomass to prepare 2,5-hexanedione, comprising: contacting and reacting a biomass raw material with a hydrogenation catalyst using hydrogen gas as a hydrogen source in the biphasic solvent system according to any one of claims 1-9 to obtain 2,5-hexanedione.

11. The process according to claim 10, which is **characterized in that** during the process, no acid, and preferably no acidic salt, is added into the reaction system.

12. The system according to claim 10, **characterized in that** the weight ratio of the organic solvent to the biomass raw material ranges from 5 to 60, and preferably from 15 to 40.

13. The process according to claim 10, which is **characterized in that** the biomass raw material is one or more of cellulose, glucose, fructose, sucrose, inulin, starch, corn straw, corn cob, and sugar cane bagasse.

14. The process according to claim 10, which is **characterized in that** in the reaction system, the hydrogen pressure ranges from 0.2 MPa to 6 MPa, and preferably from 0.5 MPa to 3 MPa.

15. The process according to any one of claims 10-14, which is **characterized in that** the weight ratio of the biomass raw material to the hydrogenation catalyst is (8-0.5): 1, and preferably (4-1): 1; and/or, the reaction temperature ranges from 160°C to 240°C, and preferably from 180°C to 220°C; and/or, the reaction time ranges from 2 hours to 16 hours, and preferably from 4 hours to 12 hours.

Fig. 1

Fig. 2

## INTERNATIONAL SEARCH REPORT

International application No.

**PCT/CN2022/094002**

### A.    CLASSIFICATION OF SUBJECT MATTER

C07C 45/42(2006.01)i;    C07C 49/12(2006.01)i

According to International Patent Classification (IPC) or to both national classification and IPC

### B.    FIELDS SEARCHED

Minimum documentation searched (classification system followed by classification symbols)

C07C

Documentation searched other than minimum documentation to the extent that such documents are included in the fields searched

Electronic data base consulted during the international search (name of data base and, where practicable, search terms used)

CNABS; CNTXT; ENTXTC; VEN; ENTXT; CNKI; ISI-Web of science: 超星, CHAOXING; 万方, WANFANG; PATENTICS: 中国石油化工股份有限公司, 己二酮, 丙酮基丙酮, 双丙酮, 氢化, 加氢, 催化剂, 有机相, 水相, 两相, 双相, 金属卤化物, 卤代盐, 卤化盐, 卤素盐, 溴化钠, 溴化钾, 氯化钾, 氯化钠, kbr, nabr, KCL, NACL, 钌, 铂, 钯, Ru, Pt, Pd, 生物质, 纤维素, +hexanedione, diacetone, hydrogenat+, halogen salt+, halide+, sodium bromide, potassium bromide, potassium chloride, sodium chloride, ruthenium, platinum, palladium, organic phase, aqueous phase, biphasic

### C.    DOCUMENTS CONSIDERED TO BE RELEVANT

| Category* | Citation of document, with indication, where appropriate, of the relevant passages | Relevant to claim No. |
|---|---|---|
| Y | CN 109896938 A (DALIAN INSTITUTE OF CHEMICAL PHYSICS, CHINESE ACADEMY OF SCIENCES) 18 June 2019 (2019-06-18) description, paragraphs [0009]-[0020] | 1-15 |
| Y | CN 107445925 A (EAST CHINA UNIVERSITY OF SCIENCE AND TECHNOLOGY) 08 December 2017 (2017-12-08) description, paragraphs [0006]-[0014] | 1-15 |
| Y | CN 111218308 A (DALIAN INSTITUTE OF CHEMICAL PHYSICS, CHINESE ACADEMY OF SCIENCES) 02 June 2020 (2020-06-02) description, paragraphs [0012]-[0025] | 1-15 |
| A | CN 112047907 A (ZHEJIANG UNIVERSITY) 08 December 2020 (2020-12-08) entire document | 1-15 |
| A | CN 105439836 A (SHANXI INSTITUTE OF COAL CHEMISTRY, CHINESE ACADEMY OF SCIENCES) 30 March 2016 (2016-03-30) entire document | 1-15 |

☑ Further documents are listed in the continuation of Box C.          ☑ See patent family annex.

| | | |
|---|---|---|
| * | Special categories of cited documents: | |
| "A" | document defining the general state of the art which is not considered to be of particular relevance | "T"  later document published after the international filing date or priority date and not in conflict with the application but cited to understand the principle or theory underlying the invention |
| "E" | earlier application or patent but published on or after the international filing date | "X"  document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"  document member of the same patent family |

| Date of the actual completion of the international search | Date of mailing of the international search report |
|---|---|
| **14 July 2022** | **08 August 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

# EP 4 342 875 A1

**INTERNATIONAL SEARCH REPORT**

| International application No. |
|---|
| **PCT/CN2022/094002** |

**C.    DOCUMENTS CONSIDERED TO BE RELEVANT**

| Category* | Citation of document, with indication, where appropriate, of the relevant passages | Relevant to claim No. |
|---|---|---|
| A | CN 106220485 A (WU QIQI) 14 December 2016 (2016-12-14)<br>entire document | 1-15 |
| PX | CN 113968776 A (DALIAN INSTITUTE OF CHEMICAL PHYSICS, CHINESE ACADEMY OF SCIENCES) 25 January 2022 (2022-01-25)<br>description, paragraph [0020] | 1-9 |

Form PCT/ISA/210 (second sheet) (January 2015)

17

**INTERNATIONAL SEARCH REPORT**
Information on patent family members

| International application No. |
| --- |
| **PCT/CN2022/094002** |

| Patent document cited in search report | | | Publication date (day/month/year) | Patent family member(s) | | | Publication date (day/month/year) |
| --- | --- | --- | --- | --- | --- | --- | --- |
| CN | 109896938 | A | 18 June 2019 | None | | | |
| CN | 107445925 | A | 08 December 2017 | CN | 107445925 | B | 14 August 2020 |
| CN | 111218308 | A | 02 June 2020 | None | | | |
| CN | 112047907 | A | 08 December 2020 | None | | | |
| CN | 105439836 | A | 30 March 2016 | CN | 105439836 | B | 25 July 2017 |
| CN | 106220485 | A | 14 December 2016 | None | | | |
| CN | 113968776 | A | 25 January 2022 | None | | | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 105693486 A
- CN 105348056 A
- CN 101423467 B
- CN 109896938 A

**Non-patent literature cited in the description**

- *Green Chemistry.,* 2016, vol. 18, 3075-3081
- *Green Chemistry.,* 2016, vol. 18, 2956-2960
- *ChemSusChem,* 2014, vol. 7, 96-100
- *Applied Catalysis A: General,* 2015, vol. 504, 664-671